(19) Europäisches Patentamt
European Patent Office
Office européen des brevets

(11) **EP 4 678 116 A1**

(12) **EUROPEAN PATENT APPLICATION**

(43) Date of publication:
**14.01.2026 Bulletin 2026/03**

(21) Application number: **24306150.4**

(22) Date of filing: **09.07.2024**

(51) International Patent Classification (IPC):
*A61B 8/08* (2006.01)    *A61B 5/00* (2006.01)

(52) Cooperative Patent Classification (CPC):
**A61B 8/485; A61B 5/4244; A61B 8/085;**
**A61B 8/5223; A61B 8/5292; A61B 8/543**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(71) Applicant: **Echosens**
**75014 Paris (FR)**

(72) Inventors:
• **SANDRIN, Laurent**
**92340 Bourg-la-Reine (FR)**
• **AUDIÈRE, Stéphane**
**75012 Paris (FR)**
• **MIETTE, Véronique**
**94240 L'Hay-les-Roses (FR)**

(74) Representative: **Cabinet Camus Lebkiri**
**25 rue de Maubeuge**
**75009 Paris (FR)**

(54) **ELASTOGRAPHY DEVICE AND METHOD**

(57) An aspect of the invention relates to an elastography method implemented by an elastography device, the method comprising:
- obtaining a respiratory signal related to a respiratory activity of a subject; and
- acquiring a plurality of measurements of a mechanical property of a region of a body of the subject, the region being a part of a liver or a spleen of the subject;
wherein each measurement among at least a subset of the plurality of measurements of the mechanical property is acquired at a same moment of a respective respiratory cycle determined from the respiratory signal.

**FIGURE 4**

**Description**

## FIELD

The present invention relates to the field of elastography, for example liver or spleen elastography. More specifically, the invention relates to an elastography device and an elastography method to accurately determine a measurement of the liver stiffness or the spleen stiffness of a subject.

## BACKGROUND

Measuring liver stiffness (LS) has been shown to be a very useful tool to help health care professionals detect or characterize liver disease or damages, and more generally monitor the condition of the liver of a subject.

There are basically two kinds of elastography techniques for measuring stiffness: strain imaging and shear wave imaging. In strain imaging, a normal stress is applied to the tissue and the normal strain is measured. The Young's modulus is then determined from the applied normal stress and the measured normal strain. In shear wave imaging, a dynamic stress is applied to the tissue and shear waves created by the excitation are then followed to derive a measurement of the stiffness of the tissue. Techniques based on shear wave imaging include Point Shear Wave Elastography (pSWE), 2D Shear Wave Elastography (SWE) and 1D Transient Elastography (TE). An example of 1D Transient Elastography is the Vibration-Controlled Transient Elastography (VCTE) used in the FibroScan® system.

The VCTE-based elastography devices typically provide several measurements of the stiffness of the tissue (for example the liver or the spleen) collected at different timepoints when the operator triggers measurements (for example by pressing a button to generate a transient displacement). A unique value representative of the stiffness measurement is then derived from these measurements (for example, the final measurement may be a mean or a median of the series of measurements. To obtain a reliable measurement of liver stiffness, it is generally recommended to obtain a series of 10 stiffness measurements.

Even if this technology works well and provides very satisfying results, it is known that variability in the series of measurements can be quite large.

There are many factors that can influence the stiffness value of certain organs, such as the liver or the spleen, and which can therefore induce variability in the stiffness measurements. For example, liver stiffness measurements (LSM) or spleen stiffness measurements (SSM) are influenced by central venous pressure and more generally by hemodynamics effects, as detailed in the article "Liver Stiffness Is Directly Influenced by Central Venous Pressure" by G. Millonig et al., Journal of Hepatology, volume 52, Issue 2, February 2010, pp. 206-210.

## SUMMARY

The inventors of the present invention investigated whether other factors could explain this variability, and how such factors might be considered in the evaluation of the liver stiffness or spleen stiffness of the subject, for example to assess liver fibrosis or portal hypertension, respectively.

An aspect of the invention relates to an elastography method in which the acquisition of measurements is synchronized to particular moments of the subject's breathing.

An aspect of the invention therefore relates to an elastography method implemented by an elastography device, the method comprising:

- obtaining a respiratory signal related to a respiratory activity of a subject; and

- acquiring a plurality of measurements of a mechanical property of a region of a body of the subject, the region being a part of a liver or a spleen of the subject;

wherein each measurement among at least a subset of the plurality of measurements of the mechanical property is acquired at a same moment of a respective respiratory cycle determined from the respiratory signal.

By "mechanical property", it is meant any physical property or parameter relating to the behavior of the region of the human body when subjected to one or more mechanical stresses. For example, the mechanical property may be conventional quantities such as stiffness, elasticity, Young's modulus, shear modulus, shear wave speed, viscoelasticity, viscosity, ultrasound attenuation, speed of sound, backscatter coefficient (BSC), etc. The mechanical property may also be any value derived from one or more of these physical properties or parameters.

In the following, particular reference is made to stiffness, but aspects of the invention apply to other mechanical properties. Also, particular reference is made to the liver as a region of the body, but aspects of the invention could apply to the spleen.

By "respiratory signal", it is meant a set of data relating to a respiratory activity of the subject. In particular, this data set may include information indicating when the subject is inhaling, exhaling, and the start and end times of inhalations or expirations.

In the examples described below, the respiratory signal corresponds to a signal representing the temporal variations in the volume of the subject's chest, acquired using a respiratory belt. It will be appreciated that the invention is not limited to such a signal. For example, the respiratory signal may be acquired by an air sensor located near the subject's nose or mouth, or by a microphone analyzing noises near the subject's nose or mouth to determine whether the subject is breathing in or out. The different sensors (air sensor, microphone, ...) described herein to acquire the respiratory signal can be broadly termed "respiratory sensor(s)".

By "measurements acquired at a same moment of a respiratory cycle", it is meant measurements acquired at different respiratory cycles, at similar moments or instants in time (e.g. a specific time or time period) in the different respiratory cycles. For example, the measurements may all be acquired at the end of an expiration period.

It is understood that the moments are determined based on the respiratory signal. For example, when the respiratory signal is a signal representing the chest's diameter of the subject, the end of an inspiration may correspond to a local maximum in the respiratory signal, and an end of an expiration may correspond to a local minimum in the respiratory signal. When the respiratory signal corresponds to information provided by an air sensor, the end of an expiration corresponds to the moment when there is no more outgoing air detected at the exit of the nose and/or the mouth.

By "each measurement among at least a subset of the plurality of measurements of the mechanical property is acquired at a same moment", it is meant that not all measurements are necessarily taken at the same time in the respiratory cycle. For example, it is possible to take several measurements during the same respiratory cycle. In this case, there is a first subset of measurements taken at the same first instant of the respiratory cycle, a second subset of measurements taken at the same second instant of the respiratory cycle, a third subset of measurements taken at the same third moment of the respiratory cycle, etc. For example, a couple measurements can be acquired per respiratory cycle: one measurement at the end of the inspiration and one measurement at the end of the expiration. Therefore, the plurality of measurements comprises a subset of measurements acquired at the end of the inspiration and a subset of measurements acquired at the end of the expiration.

As detailed hereinafter, the inventors have in fact determined that there is variability in the measurements of the mechanical property which is due to the breathing of the subject. This variability is greatly reduced when the measurements are acquired at the same time of the respiratory cycle.

Furthermore, depending on the point in the respiratory cycle at which the measurements are taken, different properties of the examined body part can be inferred. For example, measurements taken at the end of an expiration are much more correlated with fibrosis, while measurements taken at the end of an inspiration are more representative of a cardiovascular state. Thus, the moments can be chosen according to the diagnostic information targeted.

A benefit of the elastography method according to an aspect of the invention is an improvement in the precision of the measured values, and therefore in the diagnostic quality of these values. Another benefit is that the elastography method according to an aspect of the invention does not require the subject to hold their breath (it is even desirable that the subject breathes normally during the procedure), which can be very uncomfortable or very complicated, particularly for subjects with obesity problems or respiratory pathologies.

For example, the respiratory signal may be obtained by a respiratory sensor and the plurality of measurements of the mechanical property may be acquired by using an elastography probe of an elastography device.

In one or more embodiments, each measurement of the plurality of measurements of the mechanical property may be acquired at a same moment of a respective respiratory cycle determined from the respiratory signal.

In other words, in these embodiments, all the measurements are taken in different respiratory cycles (i.e. there is at most one measurement per respiratory cycle), and at similar moments of their respective respiratory cycles (for example, at the end of the expiration).

In one or more embodiments, the plurality of measurements of the mechanical property may be partitioned into a plurality of subsets of measurements, each subset of measurements comprising measurements acquired at a respective moment of the respiratory cycle, the moments of the respiratory cycle associated with different subsets of measurements being different from each other.

In these embodiments, several measurements are taken during the same respiratory cycle, at respective specific times. Thus, the plurality of measurements comprises a first subset of measurements taken at similar first moments of their respective respiratory cycles (for example at the end of inspiration) and a second subset of measurements taken at similar second moments of their respective respiratory cycles. respective respiratory cycles (for example at the end of expiration).

In one or more embodiments, the method may further comprise, for each measurement of the plurality of measurements:

- detecting an event in the respiratory signal, the event corresponding to a beginning of an inspiration, an end of an inspiration, a beginning of an expiration, or an end of an expiration; and

- acquiring the measurement upon the detection of the event

It is understood that in the case where there are several measurements per respiratory cycle, the measurements of the same respiratory cycle can be acquired upon detection of respective events, which may or may not be the same.

For example, each measurement of the plurality of measurement may be acquired at a respective time corresponding to a time at which the corresponding event related to the cardiac signal is detected.

In these embodiments, the acquisitions of measurements are synchronized with the detection of the corresponding events.

Alternatively, each measurement of the plurality of measurement may be acquired at a respective time corresponding to a time at which the corresponding event related to the cardiac signal is detected delayed by a predefined temporal offset.

In these embodiments, the acquisitions of measurements are delayed compared to the detection of the corresponding events.

In one or more embodiments, the method may further comprise: determining, from the plurality of measurements, a feature related to the mechanical property.

As mentioned above, acquiring the measurements at same moment(s) of the respiratory cycle reduces the variability between the measurements and therefore increases the accuracy of the evaluation of a feature relating to the mechanical property from the acquired measurements.

For example, the feature may be a function of a maximum, a minimum, a mean, a standard deviation and/or a percentile of the measurements.

In one or more embodiments, the feature may further be function of at least one value of the respiratory signal.

Another aspect of the invention relates to an elastography system comprising an electronic unit configured to:

- obtain a respiratory signal related to a respiratory activity of a subject; and

- acquire a plurality of measurements of a mechanical property of a region of a body of the subject, the region being a part of a liver or a spleen of the subject;

wherein each measurement among at least a subset of the plurality of measurements of the mechanical property is acquired at a same moment of a respective respiratory cycle determined from the respiratory signal.

It is noted that aspects of the present invention may be implemented as part of any elastography technique.

For example, the above method may be implemented in the context of Transient Elastography techniques, including ARFI (Acoustic Radiation Force Impulse), SWE (Shear Wave Elastography), TE (Transient Elastography) or VCTE (Vibration-Controlled Transient Elastography). Therefore, in one or several embodiments, the elastography device may comprise:

- a probe that comprises: a protruding part to be applied against the body of the subject and at least one ultrasound transducer;

wherein, for determining each of the measurements of the mechanical property, the electronic unit may be adapted to:

- deliver to the body of the subject, via the protruding part, a transient, low frequency mechanical pulse;

- upon delivery of said mechanical pulse, control the ultrasound transducer to emit a sequence of ultrasound pulses, and acquire echo signals received in response by the ultrasound transducer to track how a low frequency elastic wave induced by the mechanical pulse propagates through the region of the body of the subject;

- determine the respective measurement of the mechanical property related to low frequency elastic wave propagation.

In particular, the elastography device may be a VCTE device. In this case, the probe may further comprise a low frequency vibrator arranged to move the protruding part of the probe, and the delivery of the transient, low frequency mechanical pulse may comprise:

- control, by the electronic unit, the low frequency vibrator to deliver to the body of the subject said transient, low frequency mechanical pulse.

By "transient pulse", it is meant a mechanical vibration that is temporary. The duration of the pulse, that is the active time, during which there is a substantial motion protruding part (induced by the vibrator in the case of a VCTE device) is followed by a downtime during which there is no or substantially no motion of the protruding part. By substantially no motion, it is meant for instance that, during this downtime, the displacement of the protruding part that may be induced by the vibrator remains smaller than 1/10 or even 1/20 of the peak displacement of protruding part. For the transient pulses mentioned above, an actuation ratio, equal to the pulse's active time, divided by the sum of this active time and the following down time, is typically below 50%, or even below 20%. The downtime is the duration between the end of the active time and a subsequent significant motion of the protruding part (corresponding for instance to a subsequent transient, mechanical pulse), should there be any.

By low frequency pulse, it is meant that the central frequency of the pulse is below 500 Hz, or even below 250 Hz and for example greater than 20 Hz. The central frequency of the pulse is, for instance, the average or the median frequency of the spectrum of the displacement or of the speed of displacement corresponding to that pulse, or the peak frequency of a main peak of this spectrum, or the mean of the -3 dB, -6 dB or -20 dB cutoff frequencies of the spectrum.

In one or more embodiments, when the elastography device is a VCTE device, a peak-to-peak amplitude of a displacement of the protruding part of the probe induced by the low frequency vibrator may be between 0.2 and 10.0 mm, for example between 0.5 and 3.0 mm.

Yet another aspect of the invention relates to an elastography system further comprising a respiratory device configured to acquire the respiratory signal.

The respiratory device may use any technique to obtain the respiratory signal. Also, it is understood that the respiratory device may be external to the elastography device or may be part of the elastography device.

Yet another aspect of the invention relates to a non-transitory computer readable storage medium, having stored thereon a computer program comprising program instructions, the computer program being loadable into a data-processing unit and adapted to cause the data-processing unit to carry out the steps of the elastography method when the computer program is run by the data-processing device.

Other features and benefits of the method and apparatus disclosed herein will become apparent from the following description of non-limiting embodiments, with reference to the appended drawings.

## BRIEF DESCRIPTION OF THE DRAWINGS

Aspects of the present invention are illustrated by way of example, and not by way of limitation, in the figures of the accompanying drawings, in which like reference numerals refer to similar elements and in which:

- Figure 1 represents the variations of an LSM signal and a respiratory signal over time;

- Figures 2a, 2b and 2c illustrate several embodiments for acquiring measurements upon detection of specific moments in the respiratory signal;

- Figure 3 is a flow-chart describing a method for measuring a mechanical property of a region of the body of a subject according to an embodiment of the invention;

- Figure 4 schematically represents an example of an elastography device according to one or more embodiments of the invention;

- Figure 5 represents a sequence of mechanical and ultrasound pulses emitted by the elastography device of Figure 4 according to one or more embodiments of the invention;

- Figure 6 schematically represents an electronic unit of an elastography device according to one or more embodiments of the invention.

## DETAILED DESCRIPTION

One or more aspects of the invention pertains to an elastography method and device constructed and arranged to provide accurate measurements regardless of variables that have an impact on the measurement, such as operator experience, patient respiration, or blood pressure.

The inventors of the present invention have investigated the influence of respiration on the values of liver stiffness measurement (LSM) or spleen stiffness measurements (SSM).

During the respiratory cycle, abdominal pressure varies. This variation is even greater in the case of abdominal breathing (also called diaphragmatic breathing, as opposed to thoracic breathing). These variations induce changes in the LSM or SSM measurements. To better understand these changes, the inventors simultaneously measured both LSM and respiratory signals.

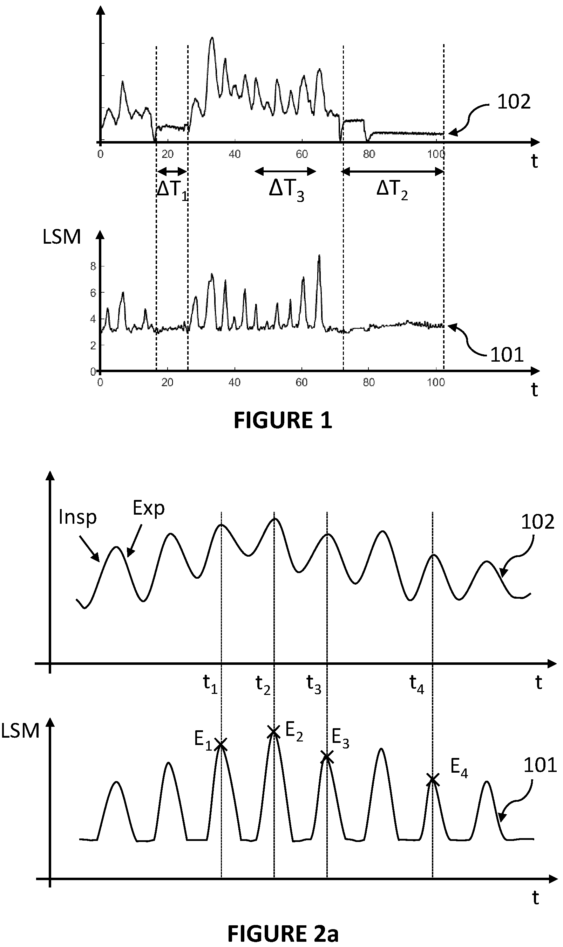

Figure 1 illustrates the results obtained on one subject. In particular, Figure 1 represents two curves:

- a curve 101 representing the LSM signal; and

- a curve 102 representing the respiratory signal.

The LSM signal 101 was acquired using a VCTE elastography device configured to acquire several liver stiffness measurements per minute, similar to the device presented in patent application EP22305298.6 assigned to the applicant of the present patent application.

Indeed, given that the respiratory frequency of an adult is of the order of 12 to 20 cycles per minute, which means one cycle every 3 to 5 seconds, each cycle being composed of one inspiration and one expiration (in which inspiration occupies approximately one third of the respiratory cycle, and the expiration occupies approximately two thirds of the respiratory cycle), it was interesting to acquire measurements at a sufficiently high rate (at least 4 measurements per second, for example 10 measurements per second) to follow more precisely the variations of the LSM over time, and in particular during a respiratory cycle. The LSM signal 101 is expressed in Kilopascals (kPa).

The respiratory signal 102 was acquired by a respiratory belt measuring changes in chest diameter over time. This signal 102, whose amplitude depends on the subject's chest diameter, is expressed in arbitrary units. To maximize the correlation between the measurement signal 101 and the respiratory signal 102, the respiratory belt can be placed close to the region in which the mechanical property is measured.

It is understood that all the observations deduced from these curves also apply to a spleen stiffness measurement (SSM) signal. Indeed, it is known that the LSM and the SSM are correlated, and that variations in the LSM values are repercussed in the SSM values. Thus, the variations observed in the LSM signal linked to hemodynamic changes and changes in abdominal pressure are also observable in the SSM signal. Therefore, aspects of the present invention also apply to an SSM signal.

Referring back to Figure 1, it first appears from the curves 101, 102 in Figure 1 that LSM values increase during inspiration (i.e. when the respiratory signal 102 is increasing) and decrease during expiration (i.e. when the respiratory signal 102 is decreasing). Thus, the peaks of the LSM signal 101 coincide temporally with the peaks of the respiratory signal 102.

In addition, the value of the peaks (i.e. local maxima) of the LSM signal 101 depends on the volume of inspired air (the "deeper" the inspiration, the higher the value of the LSM peak), while the minimum values of the LSM signal 101 (at the end of respiratory cycle or at the start of respiratory cycle) remains relatively stable.

The relative stability of the minimum values of the LSM signal 101 is all the more remarkable since these minimum values are not affected by the minimum values of the respiratory signal 102. This can be shown in Figure 1, which represents two time intervals $\Delta T_1$, $\Delta T_2$ during which the subject voluntarily held his breath. The first time interval $\Delta T_1$ (around 20-25 seconds) is associated with a first average value of the respiratory signal 102. The second time interval $\Delta T_2$ (around 70-100 s) is associated with a second average value of the respiratory signal 102 lower than the first value (i.e. the average value of the respiratory signal 102 over time interval $\Delta T_1$). It is noted that the "gap" observed in the respiratory signal 102 during the second time interval $\Delta T_2$ (around 80 s) may be caused by a movement of the respiratory belt, or by the fact that the belt has been tightened.

Two observations can be made:

- the temporal variations of the LSM signal 101 are lower during periods of apnea $\Delta T_1$, $\Delta T_2$ (the peaks are lower than when the subject is breathing); and

- the average value of the LSM signal 101 is approximately the same over the two periods of apnea $\Delta T_1$, $\Delta T_2$, and approximately corresponds to the values of the LSM signal at the end or start of a respiratory cycle.

Figure 1 also represents a third time interval $\Delta T_3$ (around 40-55 s) during which the subject is breathing: the respiration curve 102 presents rises and falls corresponding respectively to inspirations and expirations. During this third time interval $\Delta T_3$, the values of local minimums vary quite significantly. In contrast, over this same time interval, the values of the local minimums of the respiratory signal 102 vary very little.

Generally speaking, it can be observed that the minimum values of the LSM signal 102 vary relatively little around an average value RV (represented in Figure 1), independently of variations in the values of the respiratory signal 101. Conversely, the maximum values (i.e. the upward peaks) of the LSM signal 102 vary significantly, and in a manner correlated with the respiratory signal 101.

It is noted that other factors than respiration can generate variations in the LSM signal, for example hemodynamic effects. Indeed, in the not yet published patent application EP24305031.7 assigned to the same applicant, the inventors noticed that variations in the cardiac signal (in particular the components of the CVP waveform) can be observed in the LSM signal.

From these observations, the inventors of the present invention arrived at the following conclusion: as the liver stiffness measurement (LSM) signal fluctuates in sync with the respiratory cycle, it is beneficial to acquire liver stiffness measurements at specific points during respiration (for example, taking a measurement at the beginning of each inhalation). Such synchronization of the acquisition of liver stiffness (LS) measurements makes it possible to reduce the variability of the measurements. Indeed, it appears from Figure 1 that if the measurements are acquired at the same moments of the different respiratory cycles, their variability is much lower than if these measurements are carried out randomly, as it is conventionally done with existing elastography devices (in which case, a measurement can correspond to a high peak, another at a low peak, another at a median value, etc. and the variability of these measurements can thus become very high).

It is noted that an alternative would be to calculate the different measurements when the subject is in apnea. However, this involves unpleasant constraints for the subject (who must hold his breath several times). Additionally, depending on when the subject holds their breath (for example, at the end of an inspiration or at the end of an expiration), the average values of the respiratory signal during apnea periods are different, as are the values of the abdominal pressure. This may have an impact on the measurement of LS (and therefore generate variability in the measurements, which reduces their diagnostic value).

An aspect of the present invention can beneficially be carried out while the subject is breathing, thus making the examination less unpleasant for the subject, and allowing a more precise evaluation of the measurement.

In one or more embodiments, a single measurement is acquired during a respiratory cycle, and all measurements are acquired at the same time of their respective respiratory cycle. For example, each measurement may be acquired at the beginning or the end of an inspiration, or at the beginning or the end of an expiration.

It is noted that on the curve of the respiratory signal 102 in Figure 1, an inspiration corresponds to an ascending portion of the curve 102, and an expiration corresponds to a descending portion of the curve 102. Therefore, the acquisition of the measurement may be triggered upon detection of an upward peak (when the measurement is acquired at an end of an inspiration or the beginning of an expiration) or a downward peak (when the measurement is acquired at an end of an expiration or the beginning of an inspiration) in curve of the respiratory signal 102.

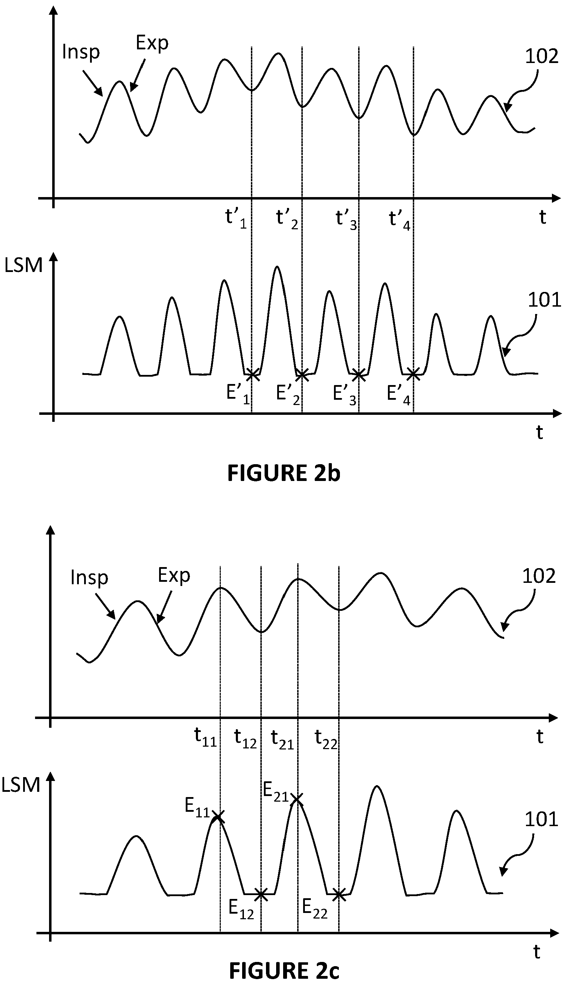

Such embodiments are represented in Figures 2a and 2b. Figures 2a and 2b represent a schematic, simplified version of the curves 101, 102 of Figure 1. As in Figure 1, curve 101 represents the LSM time signal, and curve 102 represents the respiratory time signal.

As mentioned above, a phase during which the respiratory signal 102 is increasing corresponds to an inspiration ("Insp" in Figures 2a and 2b) and a phase during which the respiratory signal 102 is decreasing corresponds to an expiration ("Exp" in Figures 2a and 2b).

In the example of Figure 2a, the measurements $E_1$, $E_2$, $E_3$, $E_4$ are acquired at different times $t_1$, $t_2$, $t_3$, $t_4$ corresponding to ends of inspiration (or, approximately equivalently at times corresponding to beginnings of expiration). In this example, the detection of an upward peak (i.e. a local maximum) in the curve of the respiratory signal 102 therefore triggers the acquisition of a corresponding LS measurement $E_1$, $E_2$, $E_3$, $E_4$.

In some embodiments, a measurement is acquired at each upward peak in the respiratory signal 102. However, this is not mandatory, and it is possible that at certain upward peaks, no measurement is acquired - this is particularly the case in the example shown in Figure 2a, in which no measurement is taken during of the intermediate peak between times $t_3$ and $t_4$. For example, it is possible that at certain peaks in the respiratory signal, the elastography device is not ready to acquire a measurement.

In the example of Figure 2b, the measurements $E'_1$, $E'_2$, $E'_3$, $E'_4$ are acquired at different times $t'_1$, $t'_2$, $t'_3$, $t'_4$ corresponding to beginnings of inspiration (or, approximately equivalently at times corresponding to ends of expiration). In this example, the detection of a downward peak (i.e. a local minimum) in the curve of the respiratory signal 102 therefore triggers the acquisition of a corresponding LS measurement $E'_1$, $E'_2$, $E'_3$, $E'_4$.

In some embodiments, a measurement is acquired at each downward peak in the respiratory signal 102. However, this is not mandatory, and it is possible that at certain downward peaks, no measurement is acquired.

The detection of an upward or downward peak in a signal is known and is not further detailed here. For example, an upward or downward peak may be detected by detecting that a rising or a falling edge of the signal reaches a predefined threshold.

It is noted that the acquisition of a measurement may be carried out at times other than at the end or the beginning of an expiration or an inspiration. For example, it is possible to acquire the measurement at the expiration of a predefined time offset following a peak (upward or downward).

Also, it is noted that in embodiments, derived measurements can be calculated from the acquired LS measurements and the corresponding respiratory signal values. For example, a derived measurement can be equal to $E_i/V_i$, where Vi corresponds to the value of the respiratory signal at the time the measurement $E_i$ is acquired, or $E_i/V$, where V is an average value of the respiratory signal over a time window associated with measurement $E_i$, etc.

In alternative embodiments, several measurements may be acquired during a same respiratory signal. For example, two measurements may be acquired during one respiratory cycle: one measurement acquired at the end of the expiration (or the beginning of the inspiration) of the respiratory cycle, and one measurement acquired at the end of the inspiration (or the beginning of the expiration) of the respiratory cycle.

Such embodiment is represented in Figure 2c. In the example of Figure 2b, the measurements $E_{11}$, $E_{12}$, $E_{21}$, $E_{22}$ are acquired at different times $t_{11}$, $t_{12}$, $t_{21}$, $t_{22}$. $E_{11}$ and $E_{21}$ are acquired at respective times $t_{11}$ and $t_{21}$ corresponding to two successive ends of inspiration (or, approximately equivalently, at two successive beginnings of expiration). $E_{12}$ and $E_{22}$ are acquired at respective times $t_{12}$ and $t_{22}$ corresponding to two successive beginning of inspiration (or, approximately equivalently, at two successive ends of expiration).

It is thus possible to obtain estimations of the amplitude (i.e. the difference between the maximum value and the minimum value) of the LSM signal over several respiratory cycles. For example, these measurements may then be used to determine a value representative of the amplitude of the LSM signal. In the example of Figure 2c, two estimations of the amplitude are obtained: $a_1 = E_{11} - E_{12}$ and $a_2 = E_{21} - E_{22}$. It will be appreciated that the number of estimates (i.e. the number of respiratory cycles during which measurements are acquired) can be greater than 2, for example around five, ten or even more than ten. It is then possible to calculate for example the average, or a percentile of all the estimates obtained, to have a single value representative of the amplitude of the LSM signal 101.

Instead of using values of the LS measurements themselves, it is possible to use normalized values, taking into account the values of the respiratory signal when acquiring the measurements. For example, if $V_{ij}$ denotes the value of the respiratory signal 102 at the time the measurement $E_{ij}$ is acquired, the estimations above may be replaced by:

$$a_1 = E_{11}/V_{11} - E_{12}/V_{12} \text{ and } a_2 = E_{21}/V_{21} - E_{22}/V_{22}$$

or:

$$a_1 = (E_{11} - E_{12})/(V_{11} - V_{12}) \text{ and } a_2 = (E_{21} - E_{22})/(V_{21} - V_{22})$$

or another function of $E_{11}$, $E_{12}$, $E_{21}$, $E_{22}$, $V_{11}$, $V_{12}$, $V_{21}$ and $V_{22}$.

It will be appreciated that depending on the desired information and the medical application envisaged, it is possible to acquire either a single measurement during a respiratory cycle (always at the same moment of the cardiac cycle), or several measurements during the same respiratory cycle.

Indeed, referring again to Figure 1, the observed curves 101, 102 indicate that the upward peaks in the LSM signal 101 are mostly influenced by abdominal pressure, while local minima (or the low plateaus) of the LSM signal 101 are probably more correlated with stiffness of the parenchyma (and therefore with a level of fibrosis).

To go further, this means that the LSM signal can be seen as the combination of two components:

- a "dynamic" component reflecting the influence of pressure variations inside the part of the body for which the stiffness is measured and of respiration on the signal; and
- a "resting" component, which is not influenced by these factors, and in particular by respiration.

The resting component reflects the more static contribution from the liver parenchyma and is believed to be much more linked to the fibrosis than the dynamic component. This resting component typically corresponds to a component of the signal around the value RV in Figure 1.

Furthermore, it has be observed that, when the probe is inclined relative to a position perpendicular to the surface of the subject's body where it is applied, the measurement thus acquired is generally higher than the true value of the measurement. Thus, a lack of perpendicularity of the probe in relation to the subject's body leads to overestimates of the measurement value. The resting component also eliminates or reduces overestimations related to probe perpendicularity.

The dynamic component of the signal reflects variations due to respiration as well as the effect of blood pressure due to hemodynamic and cardiovascular characteristics, while the resting component is mainly correlated to fibrosis.

Therefore, if the LS measurements are used as part of the diagnosis of fibrosis, it may be interesting to trigger the acquisition of each measurement at a low peak of the LSM signal, i.e. at the end of expiration (or at the start of inspiration). This corresponds to the example of Figure 2a.

If the LS measurements are used as part of the diagnosis of a cardiovascular affection it may be interesting to trigger the acquisition of each measurement at a high peak of the LSM signal, i.e. at the beginning of expiration (or at the end of inspiration). This corresponds to the example of Figure 2b.

If the aim is to evaluate the hemodynamic and cardiovascular variations, it may be interesting to acquire, for a plurality of respiratory cycles, two measurements: one measurement at the high peak of the LSM signal and one measurement at the low peak, to calculate the difference between these two measurements and therefore estimate the amplitude of the LSM signal. This corresponds to the example of Figure 2c.

It will be appreciated that other applications and embodiments may be considered.

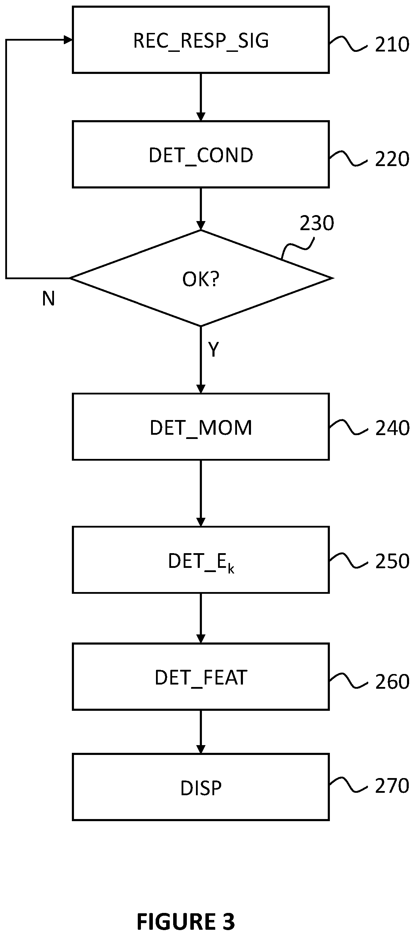

Figure 3 represents a flow-chart of a method for determining measurements of a mechanical property of a part of the liver of the spleen of the subject in one embodiment of the invention. The method of Figure 3 may be implemented by an electronic unit of an elastography device, or by an electronic unit of a computing device external to an elastography device and configured to receive data from the elastography device. The electronic unit may include one or more electronic circuitries to carry out its function(s). The one or more electronic circuitries may include a processor (e.g. a microprocessor) and a memory coded with instructions for performing the functions of the electronic unit when the instructions are executed by the processor. In an embodiment, the electronic unit may be configured to carry out the method shown in Figure 3.

According to the embodiment of Figure 3, a plurality of measurements of the mechanical property are determined at specific moments of the respiratory cycles. The determined measurements are then used for determining a feature related to the mechanical property.

In the following, the mechanical property corresponds to the tissue stiffness, and more specifically a liver stiffness. It will be appreciated that any other mechanical property may be measured, for instance the elasticity, the Young's modulus, the shear modulus, the shear wave speed, the viscoelasticity, the viscosity or any composite biomarker deriving from (or combining) one or more of the previous physical quantities. Also, it will be appreciated that the invention may also be applied to the spleen.

A respiratory signal of the subject is obtained at step 210.

The respiratory signal may be acquired, for example, by a respiratory belt or a respiratory sensor, or any other technique making it possible to detect specific moments in the respiratory cycle of the subject. In an embodiment, the respiratory signal is supplied to the electronic unit of the elastography device or a remote device in communication with the elastography device.

At an optional step 220, one or more conditions for the electronic unit to enter a measurement mode may be determined. Such measurement mode may correspond to a mode in which the electronic unit is ready to start the determination of the measurements. While the electronic unit is not in the measurement mode, no measurements is taken.

The one or more conditions may include, for example:

- a condition as to whether the position and direction of probe is adequate, i.e. whether probe is correctly positioned with respect to region of body for which measurements of the mechanical property are to be obtained. The US patent application US17/695,053 assigned to the applicant details examples of determination of such condition; and/or

- a condition as to whether tip of the probe is properly applied against body of the subject.

In an optional step 230, the electronic unit may determine whether all conditions of step 220 are met or satisfied. While at least one condition is not met or satisfied (step 220, arrow "N"), the electronic unit does not enter the measurement mode and acquisition of the measurements $E_k$ cannot begin. If all conditions are met or satisfied (step 220, arrow "Y"), the electronic unit proceeds with the measurement mode. Once the electronic unit is in measurement mode, it may monitor the occurrence of an event related to the ECG signal.

At step 240, a specific moment (or instant, or time) of the respiratory cycle in the respiratory signal is detected by the electronic unit. As detailed above, the specific moment may correspond to an upward peak or a downward peak in the respiratory signal. The detection 240 of the moment causes the acquisition (step 250) of a respective measurement $E_k$ of the mechanical property. As detailed above, depending on embodiments, there can be one measurement per respiratory cycle or several measurements per respiratory cycle. It is understood it is not mandatory to acquire measurements for each respiratory cycle. During certain cycles, it is possible that no measurement is acquired. The at least one measurement $E_k$ may be determined (step 250) by any elastography technique.

At step 260, at least one feature representative of the mechanical property of the subject may be determined from the measurements obtained at step 250.

The feature representative of the mechanical property may be a single value determined from the obtained measurements. For example, the feature may be a function of the minimum, the maximum, a given percentile, the mean, the standard variation or any statistical indicator of the obtained measurements. The feature may also be a function of a combination of at least two statistical indicators.

In embodiments, the feature representative of the mechanical property may be determined from the obtained measurements and from additional values, for example from at least one value obtained from the respiratory signal. For example, the feature may be determined from the obtained measurements and the values of the respiratory signal at times at which the measurements have been acquired. In particular, the feature may be a function of the minimum, the maximum, a given percentile, the mean, the standard variation or any statistical indicator of values derived from the measurements of the mechanical property and the values of the respiratory signal.

As mentioned above, such feature may provide more accurate information related to the mechanical property than conventional methods. In current elastography techniques, several (e.g. 10) measurements of the stiffness are determined at respective instants that can correspond to very different moments of the respiratory cycles and therefore to very different values of the stiffness. From these measurements, a feature representative of stiffness is computed (e.g. the mean or the median of the measurements), without taking into account such variability. By considering, for example, measurements always taken at the same moment (at least approximately) of the respiratory cycle, this variability is therefore reduced.

When several measurements are acquired per respiratory cycles, intermediate values can be computed from these measurements, one intermediate value corresponding to one respiratory cycle. For example, when two values are acquired at a low peak and a high peak of a same respiratory cycle, the intermediate value may be the absolute value of the difference between the two measurements. The feature may be a single value determined from the obtained intermediate values. For example, the feature may be a function of the minimum, the maximum, a given percentile, the mean, the standard variation or any statistical indicator of the obtained intermediate values. The feature may also be a function of a combination of at least two statistical indicators.

At an optional step 270, the feature determined at step 260 can be displayed on a screen of the elastography device and/or a device in communication with the elastography device, to be used by the medical practitioner. Other information may be displayed, in alternative or in addition. For example, the signal respiratory signal may be displayed, as well as an elastogram.

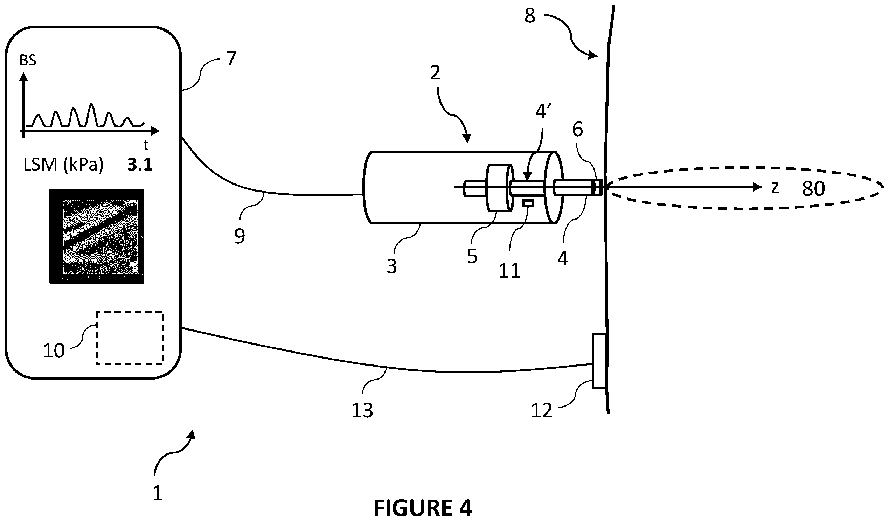

It will be appreciated that the measurements of the mechanical property of step 250 may be obtained by using any elastography technique. In particular, the measurements of the mechanical property may be acquired by an elastography device using a Vibration-Controlled Transient Elastography (VCTE) technology, such as the device described in Figure 4. It should be noted, however, that the present invention is not limited to such technology.

The elastography device 1 of Figure 4 comprises a probe 2 including a probe casing 3 (which forms the main body of the probe) to be handheld and a protruding part, which protrudes from the casing 3. The protruding part can be applied against the body 8 of the subject, to deliver mechanical pulses to it, and to transmit and acquire ultrasound (U/S) shots.

In the example of Figure 4, the protruding part is a tip 4, for instance a cylindrical tip (with a circular transducer 6 at its end).

Still, in other embodiments, the protruding part could be an ultrasound head (located at an end of the probe) including an array, for instance a linear array of U/S transducers. In this regard, it may be noted that the proposed technique can be used with a single element ultrasound transducer or with a multi-element ultrasound transducer (like an array of U/S transducers, for example a linear or convex or phased array ultrasound probe). While a single element ultrasound transducer is adapted to display A-mode and M-mode ultrasound imaging, a multi element ultrasound transducer can also display a B-mode image allowing an easier localization of the to-be-measured tissue. In the case of a multi element ultrasound transducer, at least one of the beamformed ultrasound lines is used to track how the mechanical pulses propagate. To this end, using the center beamformed ultrasound line (which is aligned with the probe axis) is beneficial, for symmetry considerations.

The probe 2 comprises also a low frequency vibrator 5, and the U/S transducer 6, which is fixed at an end of a tip 4. Here, the U/S transducer 6 plays both the role of an ultrasound emitter and the role of an ultrasound receiver (alternatively). Still, in other embodiments, the probe may comprise an U/S emitter and an U/S receiver distinct from each other. Here, the U/S transducer 6 is arranged on the axis z of the vibrator. Still, in other embodiments, the U/S transducer could be located elsewhere on the probe, not necessarily on the vibrator's axis.

The tip 4 is actuated by the low frequency vibrator 5. Here, the vibrator 5 is arranged to move the tip 4 relative to the probe casing 3. The vibrator 5 is arranged to move a shaft 4', the end of which forms the tip 4 of the probe. Still, in other embodiments, the probe may be an inertial probe. In such an inertial probe, the tip, or more generally the protruding part of the probe, could be bound to the probe casing with no motion with respect to the probe casing, the vibrator being then arranged to move a mass, inside the casing, to make the whole probe moving towards the tissue and back (by virtue of a recoil effect).

The vibrator 5 is a low frequency vibrator in that it moves the tip with a central, average frequency smaller than 500 hertz, or even smaller than 100 hertz (in contrast with ultrasound shots or echo signals, whose central frequency is typically higher than 1 megahertz, for instance between 1 and 5 megahertz). The vibrator is a low-frequency electro-mechanical actuator, for instance with one or several coils and magnets, like a loud-speaker actuator.

In this device 1, the vibrator 5 is rotationally symmetrical around a vibrator axis, which coincides with the probe axis z. When the vibrator 5 vibrates, it induces displacements that are mainly longitudinal, parallel to its axis. The shaft 4' is centered onto the axis z, and the vibrator 5 is arranged to move this shaft along the axis z.

In practice, the displacement of the ultrasound transducer 6, induced by the vibrator 5, has a peak-to-peak amplitude between 0.2 mm and 10.0 mm, and, in an embodiment, between 0.5 and 3.0 mm.

The probe 2 comprises a displacement sensor 11, arranged to output a measurement signal Sd representative of the displacement of the ultrasound transducer 6. In this embodiment, the measurement signal Sd is representative of the displacement of the ultrasound transducer 6 relative to the probe casing 3. A part of the displacement sensor 11 is fixed on the shaft 4' mentioned above while another part of the sensor is fitted in the probe, with no motion with respect to the casing 3. The displacement sensor 11 may be a Hall-effect sensor, an induction displacement sensor, or any other suitable sensor.

In one or several embodiments, the device 1 may further comprise at least one respiratory sensor 12 to be placed on the body of the subject (in the case of a chest pressure sensor for example) or near the body of the subject (in the case of a breathing sensor using a microphone or an air sensor for example - such a sensor can typically be placed near the mouth and/or nose of the subject) to record the respiratory activity of the subject and obtain a respiratory signal of the subject.

It is noted that, alternatively, the respiratory sensor 12 may not be part of the device 1, but may be connected to the device 1, for example via one or several wires, so that the device 1 may receive a signal related to respiratory activity of the subject. The device 1 may, for example, receive data acquired by the sensor and process these data to obtain the respiratory signal, or, alternatively, directly receive the respiratory signal of the subject.

The probe 2 is operatively connected to a central unit 7, which has the structure of a computer (e.g. a laptop, a smartphone, or a dedicated electronic device arranged to control and to interface the probe, and to process the signals acquired). The central unit 7 comprises at least a memory and a processor. The at least one memory is coded with machine readable instructions for carrying out function(s) of the central unit 7 when executed by the processor. The central unit 7 may also comprise a display for displaying some pieces of information, such as the curve representing the signal representative of the variations of the mechanical property of the region 80 over time during an examination of the subject, one or several elastograms, one or more features related to the mechanical property (e.g. the resting value or the dynamic value), or other pieces of information (e.g. the LSM signal). The display and graphical user interface may be controlled by the processing circuit of the central unit 7 to cause the graphical user interface to display visual information to the operator or prompt the operator of the elastography device 1 to initiate one or more actions or commands. For example, the graphical user interface may be controlled by the central unit 7 to prompt the operator of the elastography device 1 to initiate or start an elastography measurement of a mechanical property of an organ of the patient (e.g. a liver stiffness measurement or a spleen stiffness measurement) in association with the respiratory signal obtained with the respiratory sensor 12. In one or more embodiments, the processing circuit of the central unit 7 may cause the graphical user interface to display a menu that enables the operator to select and carry out specific types of measurements, such as, for example, a measurement of the mechanical property that is determined with or without the respiratory signal, and/or a measurement of the resting and/or dynamic component(s) or value(s) related to the mechanical property to provide different types of diagnostic. The processing circuit of the central unit 7 may cause the graphical user interface to display the measurement results, such as the measurement of the mechanical property that is determined with or without the respiratory signal, and/or a measurement of the resting and/or dynamic component(s) or value(s) related to the mechanical property. The central unit 7 comprises also at least one user interface, such as a keyboard, a mouse, one or more buttons and/or a touch screen to enter one or more data and/or information and/or parameters. As a non-limiting example, the additional parameters may include the age, height, weight, body mass index (BMI), fat mass index of the patient or subject.

The probe may be connected to the central unit 7 by a connection cable 9, or by a wireless link. Similarly, the respiratory sensor may be connected to the central unit 7 by a connection cable 13 or by a wireless link.

The central unit 7 may also comprise an electronic unit 10 connected to the vibrator 5, the U/S transducer 6 and the respiratory sensor 12 and configured (for instance, programmed via instruction stored in a memory) to control elastography device 1 so that it operates to acquire (or "determine") a plurality of measurements of the mechanical property of the region 80 of the body, to receive and process data obtained from the respiratory sensor 12 and to implement the method of Figure 3. The electronic unit 10 may be configured to receive the signal acquired by the respiratory sensor 12.

In one or more embodiments, the plurality of measurements of the mechanical property may be acquired in response to a manual triggering by the operator. This manual triggering may be achieved by actuating a pushbutton switch arranged on the probe casing 3, or by actuating a footswitch, for instance.

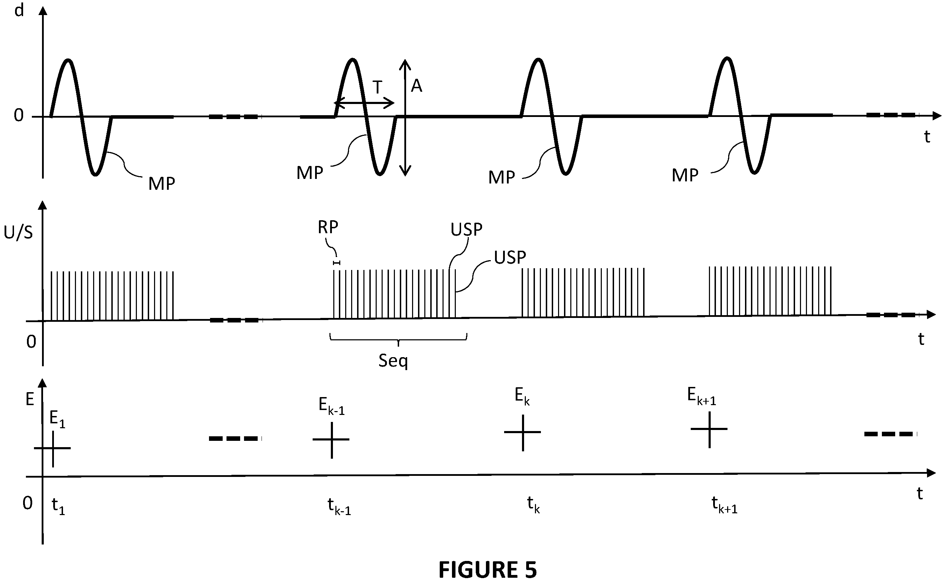

As represented in Figure 5, to obtain measurements of the mechanical property, the electronic unit 10 may be configured to control the low frequency vibrator 5 to deliver to the body 8 of the subject a plurality of mechanical pulses MP, each pulse MP being a transient, low frequency mechanical pulse. Each mechanical pulse MP corresponds to a transient displacement d of the shaft 4' along the axis z directed towards the subject's body (see Figure 4). The displacement d of the shaft 4' induced by the vibrator may for instance correspond to one period of a sinusoid having a duration T between 5 ms and 50 ms (i.e. a frequency between 20 Hz and 200 Hz).

The displacement d of the shaft 4', induced by the vibrator 5, has a peak-to-peak amplitude A between 0.2 mm and 10.0 mm, and in an embodiment between 0.5 and 3.0 mm.

The central frequency of each mechanical pulse MP may be between 10 Hz and 500 Hz, for example between 50 Hz and 200 Hz when the elastography device is configured to characterize the liver of patients (typically 50 Hz in this case).

As represented in Figure 5, for each mechanical pulse MP, the electronic unit 10 controls the U/S (ultrasound) transducer 6 (with the U/S pulser 41 of the U/S front end 40, among others) so that the U/S transducer 6 emits a sequence Seq of ultrasound pulses USP, and acquires echo signals received in response by the U/S transducer 6, to track how the mechanical pulse MP propagates through the probed region 80 of the body 8 of the subject, located in front of the tip 4 of the probe.

For this sequence Seq, the central frequency of each ultrasound pulse USP is comprised for instance between 0.5 and 10.0 megahertz. The ultrasound pulses of the sequence Seq may be transmitted one at a time, two successive pulses being separated by a pulse repetition period RP, this pulse repetition period being typically between 0.1 millisecond and 2 milliseconds (which corresponds to a pulse repetition frequency between 0.5 kilohertz and 10 kilohertz), and in an embodiment between 0.3 ms and 1 ms. The ultrasound pulses USP of the sequence Seq mentioned above may also be transmitted by groups, for instance by groups of two pulses (to compute correlations between the two corresponding echo signals). The two pulses of each group may be separated by duration between 50 and 200 microseconds, while the groups of pulses themselves are separated by a longer duration, for instance higher than 0.2 or 0.5 ms. It will be appreciated that other transmission sequences can also be considered in various embodiments.

The total duration of the sequence of U/S pulses Seq may be between 25 ms and 200 ms. This duration may be selected depending on the shear wave frequency and depending on the speed of propagation of the elastic wave which is the slower and depending on the depth of the region to be observed. For instance, for a shear wave frequency of 50 Hz, an 80 mm depth and a speed of propagation of 1 m/s (typical for shear waves in the liver of a subject), the sequence may have a duration of 100 ms.

Regarding the echo signals, acquired to track the propagation of the mechanical pulse considered, each of them is formed by a signal, received over time t by the U/S transducer 6 after the emission of one of the U/S pulses USP. It is more precisely the signal received within a given temporal window starting after this emission and having a given duration.

In the embodiments described herein, for each mechanical pulse MP, the electronic unit 10 determines tissue strain data, representative of tissue strain within the region 80, as a function of time t and as a function of depth z within the region 80. The tissue strain data is determined from the echo signals acquired to track how the mechanical pulse MP propagates through the region 80. When represented graphically as a function of time and depth, such tissue strain data forms an elastogram, from which a measurement of the mechanical property (e.g. the tissue stiffness) may be determined.

The tissue strain data is determined, from the echo signals, using a correlation technique or another patterning matching algorithm, to determine how portions of the tissue move under the influence of the elastic wave that is passing through it (the elastic wave being generated by the periodic mechanical vibration delivered by the system). For instance, for each couple of two successively received echo signals, the two echo signals are correlated with each other, which enables one to determine tissue displacement (namely, the tissue displacement that occurred between the two U/S pulses), as a function of depth, and at given time.

As mentioned above, for each mechanical pulse MP, or at least for several of them, respective tissue strain data may be obtained and a respective measurement ($E_1$, $E_{k-1}$, $E_k$, $E_{k+1}$ in Figure 5) of the mechanical property of the region 80 may be determined. Each determined measurement $E_n$ may be associated with a respective time $t_n$ which represents the instant at which the measurement is performed. For example, as in the embodiment represented in Figure 5, each measurement $E_n$ is associated with a respective time $t_n$ equal to the time at which the corresponding mechanical pulse MP has been emitted. Other embodiments are possible. For example, each measurement $E_n$ may be associated with a respective time $t_n$ equal to the time at which the last of the echo signals is acquired in response to the emission of the corresponding sequence Seq of U/S pulses USP. Alternatively, each measurement $E_n$ may be associated with a respective time $t_n$ equal to the time at which the measurement $E_n$ is determined (i.e. after the determination of the corresponding tissue strain data). It is noted that the values of the times tn obtained by all these embodiments differ very little from each other, and that this difference has little impact on the interpretation of the signal E(t) obtained.

The mechanical property of the tissue, related to low frequency shear wave propagation may be a quantity related to the tissue stiffness, such as the propagation speed of shear waves $V_s$, the shear modulus of the tissue or the Young's modulus E of the tissue (which can be derived from the slope of the stripes identified in the elastogram, or from the variation of the time of flight of the measurement pulse as a function of depth). It may also be a quantity related to low frequency shear wave attenuation in the tissue, like viscosity.

As mentioned above, it is noted that, even the elastography device represented in Figure 4 is a VCTE device, aspects of the present invention can be generalized to any type of elastography device. In particular, the present invention may be applied to other TE (Transient Elastography) devices, for example devices implementing ARFI (Acoustic Radiation Force Impulse) or SWE (Shear Wave Elastography) technologies. In ARFI and SWE, unlike in the VCTE technology, a longitudinally moving shear wave is not generated by an external vibrator. Instead, in ARFI, pressure radiation generated by ultrasounds is focused in a region of interest. The radiation pressure generates a laterally moving shear wave that is tracked outside the region of interest using ultrasound tracking pulses.

It is noted that, in the embodiments described above with reference to Figures 2a and 2b, there is no constraint as to the speed of acquisition of measurements by the elastography device. Indeed, the measurements are acquired at specific moments of the respiratory cycle, at a frequency of at most one measurement per respiratory cycle (it being understood that it is possible not to acquire measurements at certain cycles).

In the embodiments described above with reference to Figure 2c, the elastography device is preferably able to acquire at least two measurements per respiratory cycle (even if this constraint can be lifted, for example, by considering a pair of values including the value at the end of inspiration of a respiratory cycle and the value at the end of expiration of the following respiratory cycle). Knowing that the average duration of the respiratory cycle of an adult is around 3 seconds, this means that the elastography device is configured acquire one measurement each 1.5 s.

It will be appreciated that for all embodiments, the elastography device can be configured to acquire measurements at a much higher frequency, for example several measurements per second. In particular, the elastography device can be configured to acquire at least 4 measurements per second, for example 5 measurements, 10 measurements or even more than 10 measurements per second. An example of such elastography device is provided for example in patent EP 4 245 224 assigned to the applicant.

Such high acquisition rate elastography devices make it possible, for example, to obtain a signal representing variations in the measurement of the mechanical property, such as the LSM signal 101 shown in Figure 1.

With such elastography devices, it is possible, for example, to continuously acquire measurements (i.e. to perform acquisitions at a predefined rate, for example 5 or 10 measurements per second), to obtain, from the measurements thus acquired, the signal representing the temporal variations in the measurement of the mechanical property, and to determine, from the signal obtained, measurements corresponding to specific moments of the respiratory cycle (i.e. the measurements of step 250 of Figure 3).

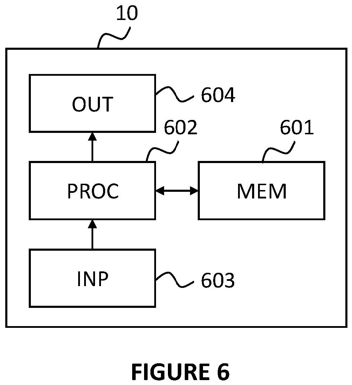

Figure 6 schematically represents an example of an electronic unit 10 of an elastography device configured to implement the method of Figure 3.

The electronic unit 10 can form at least part of a computer, and includes a non-transitory memory 601 to store program instructions loadable into a circuit computer 602 (e.g. a microelectronic circuit or circuitry) and adapted to cause the computer circuit 602 to carry out one or several steps of the method(s) or function(s) of the device(s) described herein when the program instructions are executed by the computer circuit 602.

The memory 601 may also store data and useful information for carrying the steps of the method(s) described herein. The circuit 602 may be for instance:

- a processor or a processing unit adapted to interpret instructions in a computer language, the processor or the processing unit may comprise, may be associated with or be attached to a memory comprising the instructions, or

- the association of a processor / processing unit and a memory, the processor or the processing unit adapted to interpret instructions in a computer language, the memory comprising said instructions, or

- an electronic card wherein the steps of the invention are described within silicon, or

- a programmable electronic chip such as a FPGA chip (for « Field-Programmable Gate Array »).

This electronic unit 10 further comprises an input interface 603 for receiving data related to LS measurements and data related to a respiratory signal and an output interface 604 for providing at least a feature determined in step 260 of Figure 3.

Optionally, the computer circuit 602 may be connected to a screen and may be configured to control the screen and the graphical user interface to cause the graphical user interface to display predetermined information such as, for example, the curve of the respiratory signal, value of the determined feature, etc.

Expressions such as "comprise", "include", "incorporate", "contain", "is" and "have" are to be construed in a non-exclusive manner when interpreting the description and its associated claims, namely construed to allow for other items or components which are not explicitly defined also to be present.

Reference to the singular is also to be construed in be a reference to the plural and vice versa.

A person skilled in the art will readily appreciate that various parameters disclosed in the description may be modified and that various embodiments disclosed may be combined without departing from the scope of the invention.

**Claims**

1.  An elastography method implemented by an elastography device, the method comprising:

    - obtaining a respiratory signal related to a respiratory activity of a subject; and
    - acquiring a plurality of measurements of a mechanical property of a region of a body of the subject, the region being a part of a liver or a spleen of the subject;

    wherein each measurement among at least a subset of the plurality of measurements of the mechanical property is acquired at a same moment of a respective respiratory cycle determined from the respiratory signal.

2. The elastography method of claim 1, wherein each measurement of the plurality of measurements of the mechanical property is acquired at a same moment of a respective respiratory cycle determined from the respiratory signal.

3. The elastography method of claim 1, wherein the plurality of measurements of the mechanical property is partitioned into a plurality of subsets of measurements, each subset of measurements comprising measurements acquired at a respective moment of the respiratory cycle, the moments of the respiratory cycle associated with different subsets of measurements being different from each other.

4. The elastography method of any one of the preceding claims, further comprising, for each measurement of the plurality of measurements:

  - detecting an event in the respiratory signal, the event corresponding to a beginning of an inspiration, an end of an inspiration, a beginning of an expiration, or an end of an expiration; and
  - acquiring the measurement upon the detection of the event.

5. The elastography method of claim 4, wherein each measurement of the plurality of measurement is acquired at a respective time corresponding to a time at which the corresponding event related to the cardiac signal is detected.

6. The elastography method of claim 4, wherein each measurement of the plurality of measurement is acquired at a respective time corresponding to a time at which the corresponding event related to the cardiac signal is detected delayed by a predefined temporal offset.

7. The elastography method of any one of the preceding claims, further comprising: determining, from the plurality of measurements, a feature related to the mechanical property.

8. The elastography method of claim 7, wherein the feature is a function of a maximum, a minimum, a mean, a standard deviation and/or a percentile of the measurements.

9. The elastography method of claim 7 or 8, wherein the feature is further function of at least one value of the respiratory signal.

10. An elastography system comprising an electronic unit configured to:

  - obtain a respiratory signal related to a respiratory activity of a subject; and
  - acquire a plurality of measurements of a mechanical property of a region of a body of the subject, the region being a part of a liver or a spleen of the subject;

  wherein each measurement among at least a subset of the plurality of measurements of the mechanical property is acquired at a same moment of a respective respiratory cycle determined from the respiratory signal.

11. The elastography system of claim 10, further comprising a respiratory device configured to acquire the respiratory signal.

12. A non-transitory computer readable storage medium, having stored thereon a computer program comprising program instructions, the computer program being loadable into a data-processing unit and adapted to cause the data-processing unit to carry out the steps of any of claims 1 to 9 when the computer program is run by the data-processing device.

**FIGURE 1**

**FIGURE 2a**

**FIGURE 2b**

**FIGURE 2c**

**FIGURE 3**

**FIGURE 4**

EP 4 678 116 A1

**FIGURE 5**

10

OUT ～ 604

602    601

PROC ◄──► MEM

INP ～ 603

**FIGURE 6**

Europäisches Patentamt

European Patent Office

Office européen des brevets

# EUROPEAN SEARCH REPORT

**Application Number**

**EP 24 30 6150**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2021/196235 A1 (BAE UNMIN [US] ET AL) 1 July 2021 (2021-07-01) <br> * paragraph [0001] * <br> * paragraph [0021] * <br> * paragraph [0024] * <br> * paragraph [0027] * <br> ----- | 1-12 | INV. <br> A61B8/08 <br> A61B5/00 |
| A | LING WENWU ET AL: "Assessment of impact factors on shear wave based liver stiffness measurement", EUROPEAN JOURNAL OF RADIOLOGY., vol. 82, no. 2, 1 February 2013 (2013-02-01), pages 335-341, XP093223416, NL ISSN: 0720-048X, DOI: 10.1016/j.ejrad.2012.10.004 <br> * the whole document * <br> ----- | 1-12 | |

**TECHNICAL FIELDS SEARCHED (IPC)**

A61B

The present search report has been drawn up for all claims

| Place of search | Date of completion of the search | Examiner |
|---|---|---|
| Munich | 13 November 2024 | Willig, Hendrik |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 30 6150

This annex lists the patent family members relating to the patent documents cited in the above-mentioned European search report.
The members are as contained in the European Patent Office EDP file on
The European Patent Office is in no way liable for these particulars which are merely given for the purpose of information.

13-11-2024

| Patent document cited in search report | Publication date | Patent family member(s) | Publication date |
|---|---|---|---|
| US 2021196235 A1 | 01-07-2021 | CN 111343925 A | 26-06-2020 |
| | | EP 3694418 A1 | 19-08-2020 |
| | | JP 7253540 B2 | 06-04-2023 |
| | | JP 2020536668 A | 17-12-2020 |
| | | US 2021196235 A1 | 01-07-2021 |
| | | US 2022386996 A1 | 08-12-2022 |
| | | WO 2019072552 A1 | 18-04-2019 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 22305298
- EP 24305031
- US 17695053 B
- EP 4245224 A

### Non-patent literature cited in the description

- **G. MILLONIG et al.** Liver Stiffness Is Directly Influenced by Central Venous Pressure. *Journal of Hepatology*, February 2010, vol. 52 (2), 206-210